**Europäisches Patentamt**

**European Patent Office**

**Office européen des brevets**

(11) Publication number: **0 314 066**
**A2**

## (12) EUROPEAN PATENT APPLICATION

(21) Application number: **88117752.1**

(22) Date of filing: **25.10.88**

(51) Int. Cl.⁴: **G01N 33/569 , G01N 33/548 , G01N 33/531**

(30) Priority: **28.10.87 JP 272035/87**

(43) Date of publication of application:
**03.05.89 Bulletin 89/18**

(84) Designated Contracting States:
**AT BE CH DE FR GB IT LI NL SE**

(71) Applicant: **Eisai Co., Ltd.**
**6-10, Koishikawa 4-chome Bunkyo-ku**
**Tokyo 112(JP)**

(72) Inventor: **Sawada, Takashi**
**9, 17, Sengen 2-chome**
**Tsukuba-shi Ibaraki(JP)**
Inventor: **Tohmatsu, Junichi**
**10-26, Nakatakatsu 2-chome**
**Tsuchiura-shi Ibaraki(JP)**
Inventor: **Miyoshi, Isao**
**282-20, Nakamama**
**Kochi-shi Kochi(JP)**
Inventor: **Taguchi, Hirokuni**
**292-55, Nakamama**
**Kochi-shi Kochi(JP)**

(74) Representative: **Hansen, Bernd, Dr.rer.nat. et al**
**Hoffmann, Eitle & Partner Patentanwälte**
**Arabellastrasse 4 Postfach 81 04 20**
**D-8000 München 81(DE)**

(54) **Reagent and method for assaying ATLV antibody.**

(57) An ATLV antibody is assayed in a test specimen by adding the test specimen and an absorption specimen, which has been prepared by adding ATLV antigen to said test specimen, to transblotting nitrocellulose membranes of said ATL-associated antigen respectively, and comparing the bands thus formed with each other.

EP 0 314 066 A2

# REAGENT AND METHOD FOR ASSAYING ATLV ANTIBODY

This invention relates to a reagent and a method for assaying an ATLV antibody. Thus it is available mainly in the field of medicine, in particular, in diagnostic drugs and laboratory chemicals.

( Statement of Prior Arts )

ATLV is an abbreviation of adult T-cell leukemia virus which is also called ATL virus.

ATLV and the antibody therefor, which is to be assayed according to the present invention, are described in the following literature which are employed herein as references.

1) Miyoshi, I. et al: Gann, 71:155, 1980.
2) Miyoshi, I. et al: Jap. J. Clin. Oncol., 9 (Suppl.): 485, 1979.
3) Hinuma, Y. et al: Proc. Nat. Acad. Sci., 78: 6476, 1981.
4) Miyoshi, I. et al: Gann, 73:399, 1982.
5) Miyoshi, I. et al: Lancet: 683, 1982.
6) Shimoyama, M. et al: Jap. J. Clin. Oncol. 12: 109, 1982.
7) Ohkouchi et al: Immunohaematology, Vol.3, No.5, p267, 1983.

The need of assaying an ATLV antibody, a method for preparing an ATL-associated antigen required in said assay and a reagent and method of assaying an ATLV antibody by using said antigen are described in Japanese Patent Laid-Open No. 62527/1984, which is also employed herein as a reference.

According to the above reference, transfusion donors involve ATLV carriers at a significantly high ratio. Thus there is a fear that transfusion might play an important role in the transmission of ATLV. Therefore it is known that ATLV carriers should be screened by assaying an ATLV antibody in the serum of each donor to thereby prevent the transmission of ATLV through transfusion and that a sensitive method as well as a reagent for assaying an ATLV antibody should be provided therefor.

In order to satisfy these requirements, Japanese Patent Laid-Open No. 187861/1983 has disclosed a fluorescent immunoassay method or an enzyme immunoassay method with the use of ATL-associated antigen-producing cells; while the abovementioned Japanese Patent Laid-Open No. 62527/1984 has disclosed a method for assaying an ATLV antibody by using an ATL-associated antigen obtained by treating ATL-associated antigen-producing cells with a surfactant as well as a reagent therefor.

Either of the ATL-associated antigen-producing cells and the ATL-associated antigen obtained by partially purifying the same contains various non-specific antigen components other than the ATLV antigen. Therefore they would recognize not only the ATLV antibody to be assayed but also various non-specific antibody components such as an antinuclear antibody or an anti-T cell membrane antibody present in a specimen. Accordingly the results of the assay would not always exactly reflect the presence of the ATLV antibody. For example, when the serum of a patient suffering from systemic lupus erythematosus is employed as a specimen, a positive result might be sometimes obtained, although this specimen is originally negative to the ATLV antibody. This might be caused by a reaction between an antibody component accompanying systemic lupus erythematosus and a non-specific component of the ATL-associated antigen.

In order to solve this problem, Japanese Patent Laid-Open No. 36967/1985 has disclosed an assay method which comprises separately preparing an antigen by treating an appropriate ATLV-negative cell strain such as the one obtained from a patient suffering from acute lymphocytic leukemia and judging the obtained data with the use of that obtained by using said antigen as a control. This contrast method enables prevention of the assay errors caused by the non-specific antibodies. However there remain some doubts in this invention. Namely, it is not assured that the non-specific antigen recognized by the ATL-associated antigen is immunologically the same as that originating from an ATLV-negative cell strain. Exactly speaking, these antigens are different from each other. Thus the presence or absence of the latter does not always mean the presence or absence of the former, which causes some errors in the judgement on the absence or presence of the ATLV antibody according to the method of the abovementioned invention. Under these circumstances, the present inventors have attempted to establish a method for precisely and exactly assaying an ATLV antibody and consequently found that the above purpose can be achieved by separately preparing a control specimen by adding an ATLV antigen to the test specimen to be assayed and subtracting the data of the control specimen from that of the test specimen, thus completing the invention described in Japanese Patent Laid-Open No. 249058/1985.

Subsequently, the present inventors have further conducted studies by utilizing the technical concept of the invention described in Japanese Patent Laid-Open No. 249058/1985 in order to establish a method whereby an ATLV antibody in a number of specimens in routine clinical examinations can be readily assayed.

( Summary of the Invention )

As a result of extensive studies, the present inventors have found that the presence of an ATLV antibody in a specimen can be very readily judged by preparing transblotting or transcription nitrocellulose membranes of an ATL-associated antigen which has been preliminarily fractionated by, for example, electrophoresis; adding the specimen to be assayed and an absorption specimen, prepared by adding said ATL-associated artigen to the test specimen, to the transblotting nitrocellulose membranes; and comparing the bands thus formed with each other, thus completing the present invention.

Now the present invention will be described in detail.

The ATL-associated antigen to be used in the present invention is a so-called adult T-cell leukemia-associated antigen. More particularly, it occurs in ATL-associated antigen-producing cells in the form of an antigen protein or a type C virus particle. It undergoes a specific antigen/antibody reaction with an ATLV antibody, if present. The ATL-associated antigen may be prepared by, for example, incubating human cord leukocytes together with leukemia cells collected from the peripheral blood of an ATL patient to thereby establish an antigen-producing cell strain called MT-2 and treating the MT-2 cell strain with a surfactant. The ATL-associated antigen and the treatment of the MT-2 cell strain with a surfactant are described in detail in the following literature.

8) Yoshida M., et al: Proc. Nat. Acad. Sci., 79:2031, 1982

9) Miyoshi, I. et al: Gann. 72:978, 1981

10) Miyoshi, I. et al: Nature, 294:770, 1981

11) U.S. patent 4 588 681

The ATLV antigen to be used in the present invention is an antigen derived from virus particles inducing adult T-cell leukemia and characteristic to said virus. It undergoes a specific antigen/antibody reaction with an ATLV antibody, if present. It may be prepared by concentrating a culture supernatant of ATL antigen-producing cells, isolating and purifying the ATLV and then treating the same with a surfactant. For example, the culture supernatant of MT-2 cells is concentrated and centrifuged to thereby collect ATLV, which is then divided into certain fractions by centrifuging under sucrose concentration gradient. Then fractions positive to the antigen are dialyzed to thereby give purified ATLV. The purified ATLV thus obtained is treated with sodium deoxycholate and dialyzed to thereby give the aimed ATLV antigen.

It is not always necessary that the cells employed in the preparation of the ATL-associated antigen are the same as those employed in the preparation of the ATLV antigen. However it is preferable that these antigens are prepared from the same cells, since the ATLV antigen is used in the preparation of a control specimen to be used in an assay system wherein the ATL-associated antigen is utilized.

Any treatment may be effected in order to obtain the ATL-associated antigen from ATL antigen-producing cells or to obtain the ATLV antigen from ATLV without restriction. For example, it is recommended to use a surfactant such as sodium deoxycholate, octylphenoxy polyethoxyethanol or polyoxyethylene (10) octylphenyl ether. The treatment with a surfactant is described in detail in Japanese Patent Laid-Open No. 62527/1984.

In the present invention, the ATL antigen may be fractionated by, for example, electrophoresis and then transblotted onto a nitrocellulose membrane. This treatment is known per se as the Western blot technique. For example, an ATL-associated antigen $(OD_{280}; 3.6/ml)$ is subjected to electrophoresis on a 12.5 % SDS/polyacrylamide gel of 1 mm in thickness having a specimen lane of 12 cm in length with a current of 20 mA for three hours. Then it is electrically transblotted from the polyacrylamide gel to a nitrocellulose membrane with a current of 100 mA for 14 hours. Thus various protein antigens contained in the ATL-associated antigen are separated on the nitrocellulose membrane depending on molecular weight. Among these protein antigens, those having molecular weights of 32,000 daltons, 28,000 daltons, 24,000 daltons and 19,000 daltons are known as protein antigens characteristic to ATLV and called P32, P28, P24 and P19, respectively. These protein antigens serve each as an indicator of ATLV. The following literature relates to the field as described above and are employed herein as references.

12) Haynes, B. F., S. E. Miller, T. J. Palker, J. O. Moore, P. H. Dunn, D. P. Bolognesi, and R. S. Metzgar. 1983. Identification of human T cell leukemia virus in a Japanese patient with adult T cell leukemia and cutaneous lymphomatous vasculitis.
Proc. Natl. Acad. Sci. U.S.A. 80:2054.

13) Kalyanaraman, V. S., M. G. Sarngadharan, B. Poiesz., F. W. Ruscctti, and R. C. Gallo. 1981. Immunological properties of a type-C retrovirus isolated from cultured human T-lymphoma cells and comparison to other mammalian retroviruses. J. Virol. 38:906.

14) Seiki, M., S. Hattori, Y. Hirayama, and M. Yoshida. 1983. Human adult T-cell leukemia virus : complete nucleotide sequence of the provirus genome integrated in leukemia cell DNA.
Proc. Natl. Acad. Sci. U.S.A. 80:3618.

In the present invention, a test specimen to be assayed and an absorption specimen, which is obtained by adding an ATLV antigen to said test specimen, are prepared and treated, similar to the case of the invention described in Japanese Patent Laid-Open No. 249058/1985.

The test specimen to be assayed commonly comprises a test serum, for example, a serum obtained from a transfusion blood. However the present invention is not restricted thereby and the test specimen may be in any form and derived from any source. The test specimen may be added to the assay system as such. Alternately it may be diluted with normal rabbit serum (NRS) to give an appropriate concentration prior to the assay.

It is the most convenience that the NRS and the ATLV antigen are separately introduced into test tubes and then the serum to be assayed is added to each test tube. Thus the test specimen and the absorption specimen are prepared. It is preferable that each specimen is diluted with an appropriate buffer in such a manner as to give the optimum concentration for the reaction. For example, a 0.02 M tris hydrochloride buffer (pH 7.5) containing 2 % of gelatin, 0.05 % of Tween 20, 0.9 % of NaCl and 0.1 % of NaN₃ may be employed therefor.

Each specimen is added to the abovementioned transblotting nitrocellulose membrane which has been preliminarily prepared and allowed to stand at room temperature for 60 to 90 minutes. Then the nitrocellulose membrane is washed with a washing buffer solution to thereby cease the reaction. An example of the washing buffer is a 0.01 M tris hydrochloride buffer (pH 7.5) containing 0.9 % of NaCl and 0.05 % of Tween 20.

The bands thus formed may be detected by making the bands visible through an appropriate method for detecting an antigen. For example, the enzyme-labeling indirect method, the peroxidase/antiperoxidase complex method (PAP method), the avidin/biothin-labeled peroxidase complex method (ABC method) or the antihuman Ig-binding gold colloid method may be appropriately employed therefor. The present invention is not restricted by the method thus selected.

The judgement may be conducted in the following manner.

The degree of the appearance of a band is expressed according to the following criterion:

+: obvious;

±: somewhat vague; and

-: not observed.

Then the appearance of a band corresponding to a protein antigen of a given molecular weight in the test specimen is compared with that in the absorption specimen. Thus the presence or absence of the antibody for the protein antigen is judged according to the cases a to d shown in the following Table 1.

Table 1

| Case | Test specimen | Absorption specimen | Judgement |
|------|---------------|---------------------|-----------|
| a | − | − | absent |
| b | + | + | absent |
| c | ± | − | present |
| d | + | − | present |

The accuracy of the final judgement on the absence or presence of the ATLV antibody would increase with an increase in the number of bands to be compared with each other. Therefore it is preferable that the final judgement is regarded as negative when two or more bands are selected and compared with each other and every combination suggests the absence of the ATLV antibody. In other words, the final judgement should be positive when at least one combination suggests the presence of the same. It is further preferable that two or more bands selected from among those corresponding to protein antigens having molecular weights of 32,000 daltons, 28,000 daltons, 24,000 daltons and 19,000 daltons, which are characteristic to ATLV, are combined and that the final judgement is determined based on the results thus obtained, as described above. However the present invention is not restricted thereby.

As will be shown in the Test Examples hereinafter, the assay method of the present invention, which can be conveniently carried out, gives accurate results comparable to those obtained by the EIA method. Thus an ATLV antibody-negative serum can be clearly distinguished from a positive one thereby.

The assay reagent of the present invention comprises transblotting nitrocellulose membranes of an ATL-associated antigen and an ATLV antigen as essential components. Said nitrocellulose membranes and said ATLV antigen are essential in the embodiment of the abovementioned assay method of the present invention and contribute to the achievement of the same object as the assay method does.

When the assay reagent of the present invention is formulated into a kit, the kit may further include, for example, a buffer or normal rabbit serum for dilution to thereby facilitate the assay procedure. The present invention is not restricted thereby. Similarly, it may further include various reagents for detecting antigens which are employed for the detection of bands selected from among, for example, enzyme-labeled anti-human IgG antibody, a substrate and a color developer, which are employed in the enzyme-labeled indirect method; peroxidase/antiperoxidase rabbit antibody complex (PAP complex), a substrate and a color developer, which are employed in the PAP method; biotin-binding antihuman IgG antibody, avidin-binding peroxidase, a substrate and a color developer, which are employed in the ABC method; or anti-human IgG-binding gold colloid solution and a sensitizer containing silver lacate, which are employed in the anti-human IgG-binding gold colloid method, to thereby facilitate the assay procedure. The present invention is not restricted thereby.

(Examples)

To further illustrate the present invention, the following Examples will be given.

Example 1

A 0.15 M phosphate-buffered physiological saline solution (pH 7.2) was added to 2.4 x 10⁷ of MT-2 cells and the mixture was centrifuged. Then the cells were packed. 5 ml of a veronal buffer containing 0.2 % of sodium deoxycholate (pH 7.5, $\mu$ = 0.145) was added thereto. The resulting mixture was stirred with a stirrer at 4°C for four hours and then centrifuged at 10,000 rpm for 30 minutes. The supernatant was collected and filled into a dialysis tube. It was dialyzed therein against a 0.15 M phosphate-buffered physiological saline solution at 4°C for two days and then centrifuged at 10,000 rpm for 30 minutes. The supernatant was collected and referred to as the ATL-associated antigen-containing supernatant. The supernatant showed an $OD_{280}$ of 15/ml.

The ATL-associated antigen-containing supernatant thus obtained was diluted with a 0.15 M phosphate-buffered physiological saline solution in such a manner as to give an $OD_{280}$ of 3.6/ml. Then the diluted supernatant was subjected to electrophoresis on a 12.5 % SDS/polyacrylamide gel of 1 mm in thickness having a specimen lane of 12 cm in length with a current of 20 mA for three hours. After the completion of the electrophoresis, proteins contained in the supernatant were electrically transblotted onto a nitrocellulose membrane with a current of 100 mA for 14 hours to thereby give a transblotting nitrocellulose membrane.

The ATLV antigen was prepared in the following manner. 3 $\ell$ of the culture supernatant of MT-2 cells was centrifuged at 10,000 rpm for 30 minutes and the supernatant was collected. Then the collected supernatant was concentrated and the concentrate was ultracentrifuged at 100,000 rpm for two hours. Thus ATLV was collected as the precipitate. This precipitate was thoroughly suspended into 4 ml of a 0.01 M tris hydrochloride buffer (pH 7.5) containing 0.15 M of NaCl, 0.002 M of EDTA and 0.1 % $NaN_3$ and centrifuged at 10,000 rpm for 30 minutes. Then the supernatant was collected as a crude virus concentrate. This crude concentrate was laminated onto 45-ml portions of sucrose concentration gradient solutions (15 to 60 % by weight) in nitrocellulose tubes and ultracentrifuged at 22,000 rpm for 15 hours with an RPS-25-2 Rotor (Hitachi). Then 50 drops (approximately 2 ml) per tube of each solution was fractionated and collected. The ATLV in each fraction was assayed by the EIA method wherein a human ATLV antibody was employed as an inhibition antibody. ATLV-positive fractions were pooled and dialyzed against a 0.01 M tris hydrochloride buffer (pH 7.5) containing 0.15 M of NaCl, 0.002 M of EDTA and 0.1 % of $NaN_3$ to thereby give 1.15 g/ml of an ATLV solution. After the completion of the dialysis, sodium deoxycholate was added thereto to give a concentration of 0.2 % and the resulting mixture was stirred at 4°C for 30 minutes. In order to remove the sodium deoxycholate, the mixture was dialyzed against a 0.01 M tris hydrochloride solution (pH 7.5) containing 0.15 M of NaCl, 0.002 M of EDTA and 0.1 % of $NaN_3$ to thereby give the ATLV antigen. The protein contained in the ATLV antigen corresponded to an $OD_{280}$ of 1.23/ml and 35 ml thereof was recovered.

The transblotting nitrocellulose membranes and the ATLV antigen, each prepared above, were combined with each other to thereby give the assay reagent of the present invention.

Example 2

100 $\mu$l of normal rabbit serum (NRS) was introduced into a test tube and 100 $\mu$l of the ATLV antigen suspension obtained in Example 1 was introduced into another test tube. 50-$\mu$l portions of a test serum were further introduced into these tubes. The former was referred to as the test specimen while the latter was referred to as the absorption specimen.

Each specimen was thoroughly stirred and allowed to stand at 37°C for one hour. 50 $\mu$l thereof was diluted with a 0.02 M tris hydrochloride buffer (pH 7.5) containing 2 % of gelatin, 0.05 % of Tween 20, 0.9 % of NaCl and 0.1 % of $NaN_3$, which will be called the diluting buffer hereinafter, to give a total volume of 1.0 ml and then reacted with the transblotting nitrocellulose membrane obtained in Example 1 at room temperature for 60 minutes. After the completion of the reaction, the nitrocellulose membrane was washed with a 0.01 M tris hydrochloride buffer (pH 7.5) containing 0.9 % of NaCl and 0.05 % of Tween 20, which will be called the washing buffer hereinafter. Then a peroxidase-labeled anti-human IgG antibody (or anti-human IgM antibody) diluted to the optimum concentration was added thereto and reacted therewith at room temperature for 60 minutes. After the completion of the reaction, the nitrocellulose membrane was washed with purified water and subjected to an enzymatic reaction with the use of 4-chloro-1-naphthol (50 mg/dl) and 0.01 % of $H_2O_2$. The judgement was conducted based on the molecular weights of the bands thus formed.

Example 3

A test specimen and an absorption specimen, each prepared in the same manner as the one described in Example 2, were thoroughly stirred and allowed to stand at 4°C overnight. Then 50 $\mu$l of each specimen was diluted with the diluting buffer to give the total volume of 1.0 ml and reacted with the transblotting nitrocellulose membrane obtained in Example 1 at room temperature for 60 to 90 minutes. After the completion of the reaction, the nitrocellulose membrane was washed with the washing buffer and then reacted with an anti-human IgG (or anti-human IgM) rabbit antibody diluted to the optimum concentration at room temperature for 30 minutes. After the completion of the reaction, the nitrocellulose membrane was washed with the washing buffer and then reacted with an anti-rabbit IgG goat antibody diluted to the

6

optimum concentration at room temperature for 30 minutes. After the completion of the reaction, the nitrocellulose membrane was washed with the washing buffer and then reacted with a peroxidase/antiperoxidase rabbit antibody complex (PAP complex) diluted to the optimum concentration at room temperature for 30 minutes. After the completion of the reaction, the nitrocellulose membrane was washed with purified water and subjected to an enzymatic reaction with the use of 4-chloro-1-naphthol (50 mg/dl) and 0.01 % of $H_2O_2$. Then the judgement was conducted based on the molecular weights of the bands thus formed.

Example 4

A test specimen and an absorption specimen, each prepared in the same manner as the one described in Example 1, where thoroughly stirred and allowed to stand at 37°C for one hour. 50 $\mu$l of each specimen was diluted 20-fold with the diluting buffer and reacted with the transblotting nitrocellulose membrane prepared in Example 1 for 60 to 90 minutes. After the completion of the reaction, the nitrocellulose membrane was washed with the washing buffer and reacted with a biotin-binding antiIgG (or anti-human IgM) antibody at room temperature for 30 minutes. After the completion of the reaction, the nitrocellulose membrane was washed with the washing buffer and then reacted with avidin-binding peroxidase diluted to the optimum concentration at room temperature for 30 minutes. After the completion of the reaction, the nitrocellulose membrane was washed with purified water and then subjected to an enzymatic reaction by using 4-chloro-1-naphtol (50 mg/dl) and 0.01 % of $H_2O_2$. The judgement was conducted based on the molecular weights of the bands thus formed.

Example 5

A test specimen and an absorption specimen, each prepared by the same manner as the one described in Example 2, were thoroughly stirred and allowed to stand at 4°C overnight. 50 $\mu$l of each specimen was diluted with the diluting buffer to give a total volume of 1.0 ml and then reacted with the transblotting nitrocellulose membrane prepared in Example 1 for 60 to 90 minutes. After the completion of the reaction, the nitrocellulose membrane was washed with the washing buffer and then reacted with an appropriately diluted anti-human IgG (or anti-human IgM) antibody-binding gold colloid solution at room temperature for 30 to 60 minutes. After the completion of the reaction, the nitrocellulose membrane was washed with purified water and then equilibrated with a 0.2 M citrate buffer solution (pH 3.7). After removing the citrate buffer solution, a sensitizer containing silver lactate was added thereto and the mixture was allowed to react in the dark for 5 to 15 minutes. After the completion of the reaction, the sensitizer was discarded and a fixing agent was added. After effecting the fixing for five minutes, the membrane was washed with purified water. The judgement was conducted based on the molecular weights of the bands thus formed.

(Effects of the Invention)

To further illustrate the effects of the present invention, the following Test Example will be given.

Test Example 1

Specimen and method:

Three test sera which had been judged negative to the ATLV antibody by the EIA method and three ones which had been judged positive to the ATLV antibody thereby, i.e., six specimens in total, were employed.

Each specimen was treated in the same manner as the one described in Example 2 to thereby examine the presence or absence of the bands corresponding to P32, P28, P24 and P19. The final judgement on each specimen was conducted according to the method of the present invention.

Result:

Table 2 shows the results.

Serum No. 1 showed any band neither in the test specimen nor in the absorption specimen, which indicated that it was negative to the ATLV antibody.

Serum No. 2 showed a band corresponding to P 24 in the test specimen which remained in the case of the absorption specinmen thereof. This fact suggested that it was a non-specific reaction and thus the judgement was negative.

Serum No. 3 showed bands corresponding to P28 and P24 both in the test and absorption specimens. Thus these bands seemed to reflect a non-specific reaction and thus the judgement was negative.

Serum No. 4 showed bands corresponding to P32, P28, P24 and P19 in the test specimen, while these bands disappeared in the absorption specimen. This fact suggested a specific reaction and thus the judgement was positive. Similarly, bands observed in the test specimens of sera No. 5 and No. 6 disappeared in the absorption specimens. This fact also suggested specific reactions and thus the judgements were positive.

When the sera No. 2 and No. 3 were judged exclusively from the results of the test specimens, as in a conventional method, mistaken judgements, i.e., positive, might be given, though these sera were inherently negative to the ATLV antibody. However the correct results, i.e, they were negative to the ATLV antibody, could be obtained by comparing the results of the test specimens with those of the absorption specimens according to the present invention. Thus they could be clearly distinguished from positive ones.

Table 2

| Serum No. | 1 | | 2 | | 3 | | 4 | | 5 | | 6 | |
|---|---|---|---|---|---|---|---|---|---|---|---|---|
| EIA | – | | – | | – | | + | | + | | + | |
| Specimen | T* | A** | T* | A** | T* | A** | T* | A** | T* | A** | T* | A** |
| P32 | – | – | – | – | – | – | ± | – | ± | – | – | – |
| P28 | – | – | – | – | + | + | + | – | + | – | + | – |
| P24 | – | – | + | + | + | + | + | – | + | – | ± | – |
| P19 | – | – | – | – | – | – | + | – | ± | – | + | – |
| Judgement by the invention | – | | – | | – | | + | | + | | + | |

T*: Test specimen.

A**: Absorption specimen.

Claims

(1) A reagent for assaying an ATLV antibody in a test specimen by using an ATL-associated antigen, which comprises transblotting nitrocellulose membranes of said ATL-associated antigen and an ATLV antigen as essential components.

(2) A reagent for assaying an ATLV antibody as set forth in Claim 1, wherein said ATL-associated antigen is one derived from MT-2 cells.

(3) A reagent for assaying an ATLV antibody as set forth in Claim 1 or 2, wherein said ATLV antigen is one derived in MT-2 cells.

(4) A method for assaying an ATLV antibody in a test specimen by using an ATL-associated antigen, which comprises adding said test specimen and an absorption specimen, which has been prepared by adding ATLV antigen to said test specimen, to transblotting nitrocellulose membranes of said ATL-associated antigen respectively, and comparing the bands thus formed with each other.

(5) A method for assaying an ATLV antibody as set forth in Claim 4, wherein said bands are a combination of two or more bands selected from among those corresponding to protein antigens having molecular weights, as determined by SDS/polyacrylamide gel electrophoresis, of 32,000 daltons, 28,000 daltons, 24,000 daltons and 19,000 daltons.

(6) A method for assaying an ATLV antibody as set forth in Claim 4 or 5, wherein said ATL-associated antigen is one derived from MT-2 cells.

(7) A method for assaying an ATLV antibody as set forth in any of Claims 4 to 6, wherein said ATLV antigen is one derived from MT-2 cells.